# EUROPEAN PATENT SPECIFICATION

(11) **EP 0 479 047 B1**
(45) Date of publication and mention of the grant of the patent: **01.03.1995**
(21) Application number: 91115868.1
(22) Date of filing: 18.09.1991
(51) Int. Cl.: B01L 3/14, A61B 5/14

(54) **Apparatus and method for evacuating blood aspiration tubes**
Vorrichtung und Verfahren zur Evakuierung von Blutentnahmeröhrchen
Dispositif et méthode pour la mise sous vide de tubes pour prélèvements de sang

(30) Priority: 02.10.1990 JP 265593/90; 24.06.1991 JP 180322/90
(43) Date of publication of application: 08.04.1992
(73) Proprietor: DAIICHI KOGYO KABUSHIKI KAISHA, Sasebo-shi, Nagasaki-ken (JP)
(72) Inventor: Honda, Kiyoteru, c/o Daiichi Kogyo K.K., Nagasaki-ken (JP)
(74) Representative: Müller-Boré & Partner Patentanwälte

(56) References cited:
- EP-A- 0 061 317
- WO-A-88/07351
- US-A- 3 677 091
- US-A- 4 896 545

## Description

### BACKGROUND OF THE INVENTION

### Field of the Invention:

This invention relates to production of a blood drawing or aspiration tube which maintains vacuum conditions by medical organizations for obtaining a blood sample for blood analyses. More particularly, it relates to an apparatus for automatically evacuating a blood aspiration tube, whereby air may be automatically expelled from the interior of the stoppered blood aspiration tubes to produce a blood aspiration tube that maintains a vacuum.

### Description of Prior Art:

In medical organizations, blood samples are obtained for examination of possible diseases. Conventionally, blood samples were obtained by a syringe. More recently, blood aspirator tubes are being used in increasing numbers for blood sampling.

The blood-drawing tube is a transparent test tube which is stoppered by a rubber stopper, the inside of said tube maintained in a vacuum. For blood sampling with the aid of the blood aspiration tube, a syringe needle being pointed at both ends is introduced at one pointed end into the patient's skin while the other pointed end is introduced into the rubber stopper of the blood aspiration tube, and into the tube vacuum. Since the interior of the blood aspiration tube is maintained in a vacuum, the blood is automatically sucked into the blood aspiration tube for blood sampling.

Since blood sampling may be easily achieved by using the blood aspiration tubes, they are being used in increasing numbers by e.g. medical organizations.

To produce the above mentioned blood aspiration tube, it is necessary to create a state of vacuum inside the blood aspirator tube fitted with the rubber stopper. To create the state of vacuum inside the blood aspirator tube, a rubber stopper is applied against the blood-drawing tube within a vacuum chamber. However, the operation in the vacuum chamber necessitates the use of various devices such as a manipulator and equipment including the vacuum chamber itself, thus raising production costs.

A device for evacuating a stoppered sample collection tube which tube is inserted into said device is disclosed in WO 88/07351

### SUMMARY OF THE INVENTION

In view of the foregoing, it is an object of the present invention to provide an apparatus for automatically evacuating a blood aspirator tube whereby air may be automatically expelled from the inside of the stoppered blood aspirator tube to enable production of the blood aspirator tube, the inside of which is maintained in a vacuum state, by a relatively simplified production system.

In accordance with the present invention, there is provided an apparatus for automatically evacuating a blood aspirator tube comprising an arraying table on which the number of stoppered blood aspirator tubes to be evacuated are arrayed at a predetermined pitch, an air suction needle for expelling air from the inside of the stoppered blood aspirator tubes for evacuation, driving means for moving said air suction needle horizontally and vertically relative to said arraying table, a presser for pressing the stopper of said blood aspirator tube during vertical upward movement of said air suction needle, an air suction unit for sucking air through said air suction needle, and a controlling device for controlling the operations of said air suction needle, driving means, presser and the air suction unit for automatically expelling air from the inside of said air aspirator tube maintained in a tightly sealed state.

In operation, a number of stoppered and tightly sealed blood aspirator tubes, the insides of which are filled with air, are arrayed on an arraying table, and predetermined input conditions are inputted into the controlling apparatus. The controlling apparatus is then set into operation. The air suction needle, adapted to be movable horizontally and vertically, is moved horizontally over the stoppered blood aspirator tubes, arrayed at a predetermined pitch, until reaching a position directly above the selected stoppered blood-drawing tube.

The air suction needle then descends so that its distal end pierces through the stopper of the blood aspirator tube to enter the inside of the tightly sealed tube, where it sucks air in the aspirator tube by actuation of the air suction unit, creating a vacuum within the aspirator tube.

When the state of vacuum is established within the blood aspirator tube, the air suction unit is deactivated and the air suction needle is raised. A presser adapted for pressing the stopper of the blood aspirator tube during upward travel of the air suction needle is lowered at this time to press the stopper of the blood aspirator tube to allow the needle pierced through the stopper to entrain with the uplifted vacuum suction needle. Thus the air aspirator needle may be extricated smoothly from the stopper of the blood aspirator tube as the needle is uplifted.

The needle then is moved horizontally to a position directly above the neighboring stoppered blood aspirator tube to expel the air from its inside in a similar manner. By repetition of the above described sequence of operations, evacuation of the inside of the stoppered blood aspirator tubes may be achieved automatically.

In this manner, it is possible, with the apparatus of the present invention, to expel air automatically from the inside of the stoppered blood aspirator tube so that the blood aspirator tube, having its interior maintained in a vacuum, may be produced automatically. Since manual labor may be dispensed with, the operator may perform other jobs during production of the blood aspirator tube, thus improving operational efficiency.

Since air may be expelled automatically from the inside of the stoppered blood aspirator tube, the blood aspirator tube maintaining a state of vacuum in its inside may be produced by a relatively simple production system with outstanding practical advantages.

### BRIEF DESCRIPTION OF THE DRAWINGS

Fig. 1 is a side view showing an apparatus for automatically evacuating a blood aspirator tube according to a first embodiment of the present invention;
Fig. 2 is a plan view thereof;
Fig. 3 is a schematic view showing a controlling system for the apparatus shown in Figs. 1 and 2;
Fig. 4 is a side view showing an apparatus for automatically evacuating a blood aspiration tube according to a second embodiment of the present invention;
Fig. 5 is a front view thereof;
Fig. 6 is a plan view thereof; and
Fig. 7 is an enlarged side view showing a needle rest employed in the apparatus shown in Figs. 4 to 6.

### THE BEST MODE OF THE INVENTION

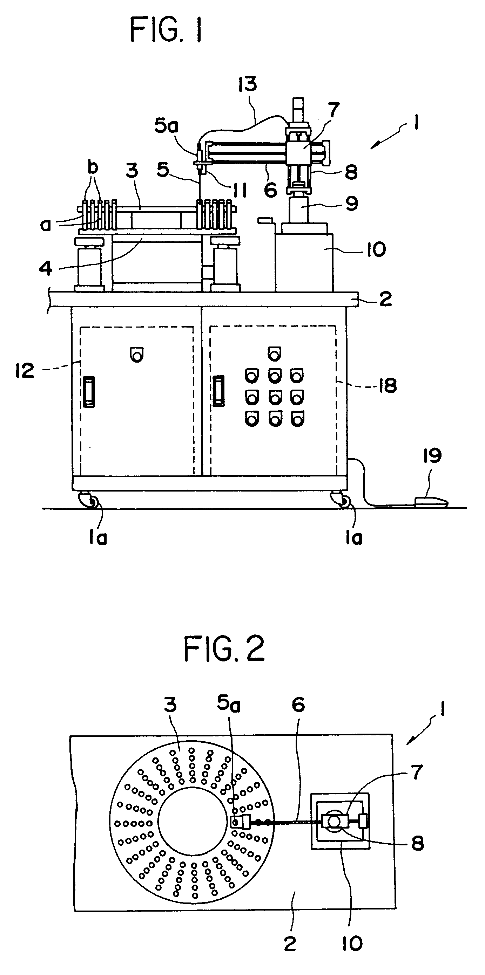

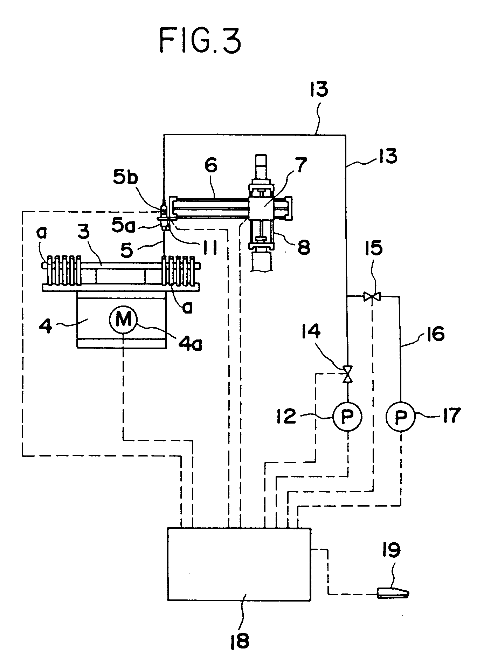

By referring to the drawings, certain preferred embodiments of the present invention will be explained in more detail. Fig. 1 is a side view of an automatic evacuating apparatus for blood aspirator tubes, Fig. 2 is a plan view showing the automatic evacuating apparatus for blood aspirator tubes shown in Fig. 1, and Fig. 3 is a schematic view showing a control system for the evacuating apparatus.

The evacuating apparatus 1 is adapted for automatically expelling air from the inside of blood aspiration tubes a, each fitted with a rubber stopper b, for producing evacuated blood aspirator tubes a.

At the middle of a table-shaped base plate 2 of the automatic evacuating apparatus for blood aspirator tubes, there is mounted an arraying table 3, on which the number of the blood aspirator tubes a to be evacuated are arrayed. A large number of blood aspirator tubes a are arrayed at predetermined intervals both circumferentially and radially towards the periphery of the disk-shaped arraying table 3. The upper open ends of the blood aspirator tubes a arrayed on the table 3 are stoppered by rubber stoppers b to hermetically seal the inside of the tubes a.

The arraying table 3 is mounted on a rotary base 4 installed on the base plate 2. An electric driving motor 4a is enclosed within the interior of the rotary base 4. By the rotational power of the driving motor, the rotary base 4 is driven rotationally to rotate the arraying table 3 mounted thereon. Although the arraying table 3 is disk-shaped in the drawing, it may be also be rectangular in contour.

An air suction needle 5 is provided over the arraying table 3 on which the blood aspirator tubes a are arrayed. The air suction needle 5 is a device for drawing air from the inside of the blood aspirator tube a stoppered by the rubber stopper b. The air suction needle 5 is similar in construction to a syringe needle and has an internal air passage along the needle axis communicating with an air suction unit 12 via air suction passage 13 which will be described later.

The air suction needle 5 is mounted with the distal end thereof directed downwards and with the upper end thereof mounted in a needle rest 5a. The needle rest 5a, which mounts the air suction needle 5, is supported for vertical movement by a distal end part of a horizontal arm 6. To the needle rest 5a, is attached a pressure sensor 5b which, when the air suction needle 5 pierces through the rubber stopper b of the blood aspirator tube a, senses the pressure acting on the air suction needle 5.

The pressure sensor 5b is connected to a controlling unit 18, which will be described later, so that data sensed by the pressure sensor 5b is transmitted to the controlling unit 18, which then discerns automatically if the air suction needle 5 has correctly pierced through the rubber stopper b of the blood aspirator tube a.

The horizontal arm 6, to which the air suction needle 5 is attached by way of the needle rest 5a, is mounted on a vertically movable base block 7 for horizontal movement.

The base block 7, on which is mounted the horizontal arm 6, is itself mounted on a vertically movable arm 8 for vertical movement. The arm 8 has its lower end supported by a supporting block 9.

The supporting block 9 has its lower end mounted on a frame 10 in turn mounted on the base plate 2.

Since the air suction needle 5 is mounted for vertical movement on the horizontal arm 6 via needle rest 5a and the horizontal arm 6 mounting the air suction needle 5 is mounted for horizontal movement on the base block 7, which is mounted so as to be movable vertically along the vertical arm 8, the air suction needle 5 is movable both horizontally and vertically.

A presser 11 is attached to a lower part at the distal end of the horizontal arm 6 to which the air suction needle 5 is mounted by means of needle rest 5a. The presser 11 acts on the rubber stopper b of the blood aspirator tube a during upward movement of the air suction needle 5 to prevent the rubber stopper b and the blood aspirator tube a, into which the air suction needle 5 has been introduced, from being moved upwards simultaneously with the air suction needle 5.

The presser 11 is extended downwards and has its lower part bent horizontally and bifurcated so that the air suction needle 5 is introduced downwards in a gap defined between bifurcated ends.

The air suction unit 12 is designed to suck air from the inside of the air aspirator tubes a by way of the air suction needle 5, and is provided below the base plate 2 of the automatic evacuator 1. The air suction unit 12 is constituted by a vacuum pump etc. and is connected to one end of an air suction conduit 13, the other end of which is connected to the air suction needle 5, so that the air suction unit 12 and the needle 5 communicate with each other through the air suction conduit 13. A vinyl hose, for example, is used as the conduit 13.

A changeover valve 14 for turning on and off the communication between the air suction needle 5 and the air suction unit 12 is provided at a connecting portion between the air suction unit 12 and the air suction conduit 13.

A second changeover valve 15 for starting and stopping the flowing out of compressed air is provided halfway via air suction conduit 13 upstream of the suction control changeover valve 14. To this changeover valve 15, is connected one end of a compressed air blowout conduit 16, the other end of which is connected to a compressed air blowout unit 17, which may be constituted by a compression pump, not shown.

The controlling unit 18 controls various operations, such as horizontal and vertical movement of the air suction needle 5, rotation of the arraying table 3, operation of the presser 11 or actuation of the air suction unit 12. By controlling these operations, the controlling unit 18 plays the role of automatically expelling air from the inside of the blood aspirator tube a tightly sealed by the rubber stopper b. The controlling unit 18 is provided adjacent to the air suction unit 12 below the base plate 2 of the automatic evacuator 1.

The air suction needle 5 is controlled in its horizontal movement and vertical movement by controlling the needle rest 5a, horizontal arm 6 and vertical arm 8, while the arraying table 3 is controlled in its rotational movement by controlling the driving motor 4a for rotary base 4.

The controlling unit 18 also controls the operation of the suction control changeover valve 14, compressed air blowout control changeover valve 15 and the compressed air blowout unit 17 etc.

A line from the pressure sensor 5b is connected to the controlling unit 18, which performs the above described controlling operation, so that data from pressure sensor 5b may be received thereby.

A foot switch 19 is adapted to turn the starting of the automatic evacuator 1 on and off by being pressed by the user's foot. The lower end of the automatic evacuator 1 is fitted with casters 1a to permit a facilitated evacuator.

The operation of the above described first embodiment will be hereinafter explained.

The rubber stopper b is fitted on the opened upper end of the blood aspirator tube a for tightly sealing the blood aspirator tube a. A number of such blood aspirator tubes a, fitted with the rubber stoppers b, are prepared. These blood aspirator tubes a are arrayed on the arraying table 3.

Additionally, a power source switch (not shown) for the automatic evacuator 1 is turned on, and an auto/manual changeover switch (not shown) is switched to the auto state. This initiates the operation of the air suction unit 12 and the controlling unit 18, while actuating the horizontal arm 6, vertical movable base block 7 and the needle rest 5a and setting the air suction needle 5 at a start position. At this time, predetermined input conditions, such as the number of the air aspirator tubes a arrayed on the table 3, are entered into the controlling unit 18.

Then, by using indication lights associated with various devices, the user can check whether these devices are operating normally or not. The indication lights indicate the normal operating state by being lit continuously or intermittently.

The compressed air blowout unit 17 is then started in order to open the compressed air blowout control changeover valve 15 to permit compressed air to flow from the unit 17 via compressed air blowout conduit 16 and the air suction conduit 13 towards the air suction needle 5 so as to be discharged at the distal end of the air suction needle 5. In this manner, it is confirmed that air does flow through the air suction needle 5, with the indication lights being lit continuously or intermittently to indicate the normal operating state of the air suction needle 5.

The compressed air blowout changeover valve 15 is then closed. After checking the indication lights to assure the various devices are in normal operating condition, the foot switch 19 is pressed by the foot to start the operation of the automatic evacuator 1.

When the foot switch 19 is actuated, the control operation of the controlling unit 18 is initiated. The horizontal arm 6, the distal end of which is attached to the air suction needle 5 via needle rest 5a, is moved forward relative to the base block 7, based on control commands from the controlling unit 18. The forward travel of the arm 6 ceases when the air suction needle 5 at the distal end of the horizontal arm 6 reaches a position directly above the selected blood aspirator tube a arrayed on the table 3.

The needle rest 5a, to which the air suction needle 5 is attached, then descends under a control command from the controlling unit 18, so that the distal end of the needle 5 abuts and pierces through the rubber stopper b of the blood aspirator tube a, with the distal end of the needle 5 intruding into the inside of the tightly sealed blood aspirator tube a. When the distal end of the air aspirator needle 5 has pierced through the rubber stopper b so as to be intruding into the interior of the blood aspirator needle 5, this state is sensed by the pressure sensor 5b of the needle rest 5a and corresponding data are transmitted to the controlling unit 18.

Based on the date from pressure sensor 5b, the controlling unit 18 discerns that the distal end of the air suction needle 5 has been normally inserted into the interior of the blood aspirator tube a, and transmits a command for opening the suction controlling changeover valve 14, which is thereby opened.

On opening of the suction controlling changeover valve 14, the air suction needle 5, which has been introduced into the interior of the blood aspirator tube a, starts its suction, so that air within the interior of the blood aspirator tube a is sucked by the needle 5 to set the state of vacuum inside the blood aspirator tube a. The air sucked by the air suction needle 5 is expelled to the outside via suction conduit 13. The indication lights are lit continuously or intermittently to indicate that an evacuation is in process.

The suction time period, for example, 2 to 3 seconds, may be suitably changed and set depending on the ability of the air suction unit 12, the inner capacity of the blood aspirator tube a, etc.

After the suction time period lapses, the controlling unit 18 issues a command for valve closure to the suction controlling changeover valve 14, which is thereby closed.

On termination of evacuation of the blood aspirator tube a, the controlling unit 18 issues a command for upward vertical movement to the needle rest 5a. Based on this command, the needle rest 5a, mounting the air suction needle 5, is raised. The presser 11, provided below the needle rest 5a, acts on the rubber stopper b of the blood aspirator tube a from above, to prevent the rubber stopper b, pierced through by the air suction needle 5, and the blood-drawing tube a, from being uplifted simultaneously with the air suction needle 5.

With the function of the presser 11, the distal end of the air suction needle 5 may be extricated freely from the rubber stopper b. The needle hole produced on extrication of the distal end of the air suction needle 5 is instantly shut due to the elasticity that is proper to the rubber material of the rubber stopper b to prevent air from flowing into the interior of the blood aspiration tube a. Thus the blood aspirator tube a with an interior maintained in a vacuum is produced.

When the needle rest 5a is raised to a predetermined height and the distal end of the air suction needle 5 completely clears the rubber stopper b, the controlling unit 18 issues a command for vertical movement to the base block 7 which then is moved upward with the horizontal arm 6 supported thereby.

As the horizontal arm 6 is raised, the air suction needle 5 and the presser 11 are similarly raised until they are halted at a predetermined height position. The horizontal arm 6 is then moved horizontally until it is halted when the air suction needle 5 reaches a position directly above the neighboring blood aspirator tube a.

A similar sequence of operations is performed under the command of the controlling unit 18 so that, by repetition of similar operations, evacuation of blood aspirator tubes a tightly sealed by rubber stoppers b are achieved automatically.

On completion of evacuation of blood aspirator tubes a in the same row, the air suction needle 5 is reverted to its starting position based on a command from the controlling unit 18. The driving motor 4a is driven under the commands from the controlling unit 18 for rotationally driving the rotary base 4. By the rotation of the rotary base 4, the arraying table 3 is similarly rotated to bring the unevacuated blood aspirator tubes a of the neighboring row to below the area of movement of the air suction needle 5. A sequence of similar evacuating operations is then performed automatically.

The controlling unit 18 operates under the input conditions to count the number of the blood aspirator tubes a arrayed on the table 3, and to expel air from the inside of all of the blood aspirator tubes a on the table 3. The operation is terminated upon completion of the evacuating operations under the preset input conditions.

### Second Embodiment

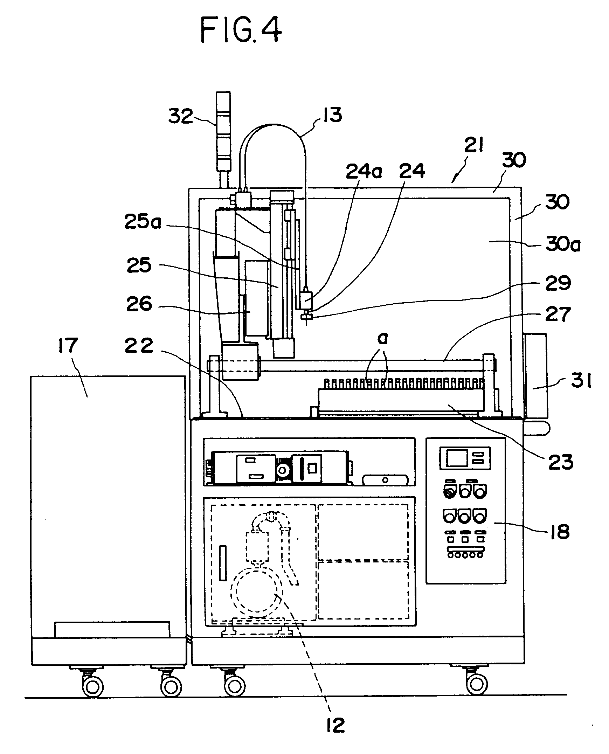

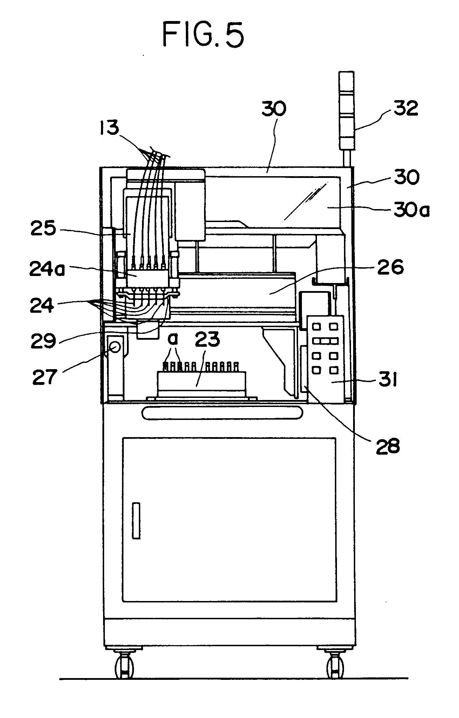

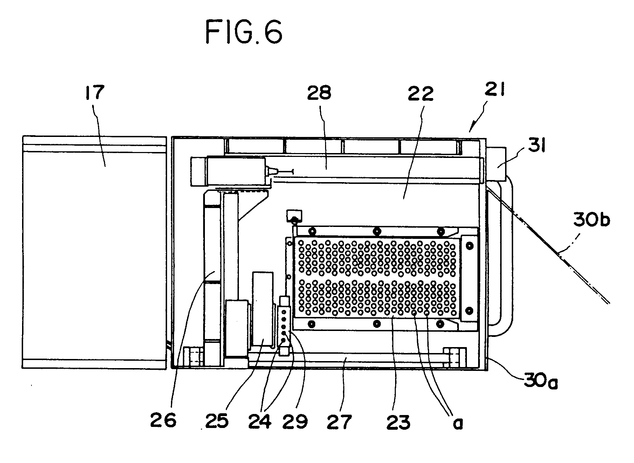

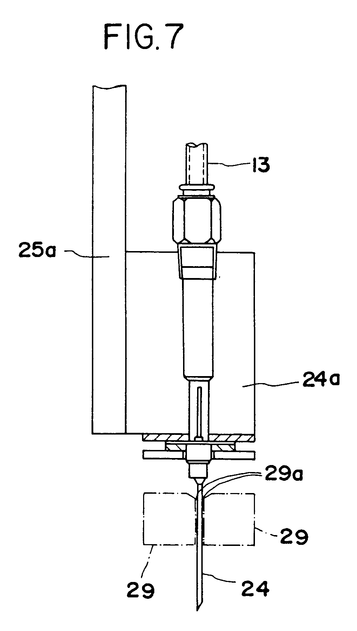

By referring to the drawings, a second embodiment of the present invention will be explained in detail. In the drawings, Fig. 4 is a side view of showing an apparatus for automatically evacuating the blood aspirator tubes, Fig. 5 is a front view of the apparatus, Fig. 6 is a plan view of the apparatus and Fig. 7 is an enlarged view showing a needle rest.

The present second embodiment differs appreciably from the preceding embodiment in that a plurality of air suction needles are provided so that a plurality of blood aspirator tubes a may be evacuated simultaneously, and in that, while the arraying table 3 of the first embodiment is rotatable, the arraying table of the present second embodiment is fixed while the air suction needles are movable in fore-and-aft and left-and-right directions over the fixed arraying table for evacuation.

Referring to these figures, showing the apparatus for automatically evacuating the blood aspirator tubes of the second embodiment, an arraying table 23, on which blood drawing tubes a to be evacuated are arrayed at a predetermined pitch, is provided near the center portion of a table-shaped base plate 22. A large number of blood aspirator tubes a are arrayed at a predetermined pitch in a latticed or staggered pattern relating to the fore-and-aft and left-and-right parameters on the arraying table 23 which, for example, may be rectangular in contour.

A plurality of air suction needles 24 are arrayed, running in left-and-right directions and mounted in their entirety on a needle rest 24a, so that the air suction needles 24 may be moved in unison by the movement of the needle rest 24a in vertical, fore-and-aft and left-and-right directions.

Since a plurality of air suction needles 24 may execute evacuation simultaneously, the evacuating ability is improved significantly over the preceding first embodiment.

The needle rest 24a, carrying a plurality of air suction needles 24, is mounted for vertical movement on an upstanding base block 25. That is, the needle rest 24a is mounted at the lower end of block 25a which is movable vertically along the upstanding base block 25. The base block 25, to which the air suction needles 24 are attached by means of the needle rest 24a and the block 25a, is mounted on a horizontal base block 26 for movement horizontally in the left-and-right direction.

The horizontal base block 26, on which the upstanding base block 25 is mounted, has both of its ends supported by a horizontal supporting arm 27 and a horizontal driving arm 28, so that the horizontal base block may be moved horizontally in the fore-and-aft direction along the horizontal supporting arm 27 and the horizontal driving arm 28 by actuation of the horizontal driving arm 28. These arms 27 and 28 are arranged horizontally along the fore-and-aft direction on the left-and-right sides on the base plate 22.

A presser 29 is attached to a lower part of the upstanding base block 25 to which the air suction needles 24 are attached by means of the needle rest 24a. The presser 29 is designed to act on the rubber stoppers b of the blood aspirator tubes a during upward movement of the air suction needles 24 to prevent the rubber stoppers b and the blood aspirator tubes a, into which the air suction needles 24 have been introduced, from being raised simultaneously with the air suction needles 24.

The presser 29 has its left and right sides supported by the vertically movable block 25a, and is formed with a plurality of needle apertures 29a, through which the air suction needles 24 are passed for downward extension.

A frame 30 is provided above the base plate 22 of the automatic evacuator 21, and a transparent plastic plate 30a is provided on the frame 30 to protect various devices provided on the base plate 22. The frame 30 is fitted with a transparent door 30b by which the blood aspirator tubes a on the base plate 22 may be introduced to or taken out of the apparatus. A switchboard 31 is mounted laterally on the door 30b.

A signal pole 32 is mounted on the top of the frame 30 and provided with red, blue and yellow indication light, to be, for example, viewed from above. The red, blue and yellow indication lights on the signal pole 32 are lit intermittently during the operation of evacuating the blood aspiration tubes a by the air suction needles 24, on termination of the operation, and during abnormal operation, respectively.

Since the construction of the present second embodiment is otherwise the same as that of the preceding first embodiment, the corresponding description is omitted for simplicity.

## Claims

1. An apparatus for automatically evacuating blood aspirator tubes comprising:
an arraying table (3; 23) on which the number of stoppered blood aspirator tubes (a) to be evacuated are arrayed at a predetermined pitch,
at least an air suction needle (5; 24) for piercing the rubber stoppers of said aspirator tubes and for expelling air from the inside of the stoppered blood aspirator tubes (a) for evacuation,
driving means (6, 7, 8; 26, 27, 28) for moving said air suction needle (5; 24) horizontally and vertically relative to said arraying table (3; 23),
a presser (11; 29) for pressing the rubber stoppers of said blood aspirator tubes (a) during vertical upward movement of said air suction needle (5; 24) out of said stoppered aspirator tubes,
an air suction unit (12) connected to said suction needle (5; 24) for sucking air through said air suction needle (5; 24), and
a controlling device (18) for controlling the operation of said air suction needle (5; 24), driving means (6, 7, 8; 26, 27, 28), presser (11; 29) and the air suction unit (12) for automatically expelling air from the inside of said blood aspirator tubes (a) maintained in a tightly sealed state.

2. The apparatus according to claim 1, further comprising compressed air flowout means (16, 17) fluidically connected to said air suction needle (5; 24) for ascertaining that the air has passed through said air suction needle (5; 24).

3. An apparatus for automatically evacuating blood aspirator tubes as claimed in claim 1 wherein the arraying table is fixed and a plurality of air suction needles (24) for expelling air from the inside of said stoppered blood aspirator tubes (a) for evacuations, movable in unison is provided so that a plurality of stoppered aspirator tubes (a) can be evacuated simultaneously.

4. A method for automatically evacuating blood aspirator tubes by using the apparatus of claim 1 comprising the steps of:
rubber stoppering a number of blood aspirator tubes (a) in an atmospheric pressure environment to tightly seal the inside of said blood aspirator tubes,
arraying a number of stoppered blood aspirator tubes (a) in a predetermined array on an arraying table (3; 23), moving at least an air suction needle (5; 24) horizontally and vertically relative to said arraying table (3; 23) to introduce a distal end thereof into the interior of one of said blood aspirator tubes (a),
sucking air from said blood aspirator tube (a),
extracting said air suction needle (5; 24) out of said blood aspirator tube (a) while pressing the stopper (b) of said blood aspirator tube, and
repeating the moving, sucking and extracting steps for each of the remaining blood aspirator tubes wherein all the above steps are carried out and controlled automatically by a control device (18).

5. A method for automatically evacuating blood aspirator tubes by using the apparatus of claim 3 comprising the steps of:
rubber stoppering a number of blood aspirator tubes (a) in an atmospheric pressure environment for tightly sealing the insides of said blood aspirator tubes,
arraying a number of stoppered blood aspirator tubes (a) in a predetermined array on an arraying table (23),
moving a plurality of air suction needles (24) horizontally and vertically relative to said arraying table (23) to introduce the distal ends thereof into the interior of the same number of said blood aspirator tubes (a),
sucking air simultaneously from said blood aspirator tubes (a),
extracting said blood aspirator needles (24) while pressing the stoppers (b) of said blood suction tubes (a), and repeating the moving, sucking and extracting steps for a plurality of sets, each consisting of said plurality of blood aspirator tubes (a), wherein all the above steps are carried out and controlled automatically by a control device (18).

## Patentansprüche

1. Vorrichtung zum automatischen Evakuieren von Blutsaugrohren mit:
einem Anordnungstisch (3; 23), auf dem eine Vielzahl von zu evakuierenden vereschlossenen Blutsaugrohren (a) unter einem vorbestimmten Intervallmuster angeordnet sind,
zumindest einer Luftsaugnadel (5; 24) zum Durchlochen der Gummistopfen der Saugrohre und zum Austreiben der Luft aus dem Inneren der verschlossenen Blutsaugrohre (a) zur Evakuation,
einer Antriebseinrichtung (6, 7, 8; 26, 27, 28) zum horizontalen und vertikalen Bewegen der Luftsaugnadel (5; 24) bezüglich des Anordnungstisches (3; 23),
einem Drücker (11; 29) zum Drücken der Gummistopfen der Blutsaugrohre (a) während der vertikalen Aufwärtsbewegung der Luftsaugnadel (5; 24) aus den verschlossenen Saugrohren,
einer Luftsaugeinheit (12), welche mit der Saugnadel (5; 24) zum Saugen von Luft durch die Luftsaugnadel (5; 24) verbunden ist, und
einer Regel- bzw. Steuereinrichtung (18) zum Regeln bzw. Steuern des Betriebes von der Luftsaugnadel (5; 24), der Antriebseinrichtung (6, 7, 8; 26, 27, 28), des Drückers (11; 29) und der Luftsaugeinheit (12) zum automatischen Austreiben von Luft aus dem Inneren der Blutsaugrohre (a), welche in einem dicht verschlossenen Zustand gehalten sind.

2. Vorrichtung gemäß Anspruch 1, welche weiterhin umfaßt eine mit der Luftsaugnadel (5; 24) fluid bzw. strömungsmäßig verbundene Druckluftausströmeinrichtung (16, 17), zum Sicherstellen, daß die Luft durch die Luftsaugnadel (5; 24) durchgeströmt ist.

3. Vorrichtung zum automatischen Evakuieren von Blutsaugrohren gemäß Anspruch 1, wobei der Anordnungstisch fixiert ist und eine Vielzahl von Luftsaugnadeln (24) zum Austreiben von Luft aus dem Inneren der mit Stopfen versehenen Blutsaugrohre (a) zum Evakuieren vorgesehen ist, welche zusammen bzw. unisono bewegbar sind, so daß eine Vielzahl von verschlossenen Saugrohren (a) gleichzeitig evakuiert werden kann.

4. Verfahren zum automatischen Evakuieren von Blutsaugrohren unter Benutzung der Vorrichtung gemäß Anspruch 1, mit den Schritten:
eine Anzahl von Blutsaugrohren (a) wird in einer Umgebung mit atmosphärischen Druck mit Gummistopfen versehen, um das Innere der Blutsaugrohre dicht abzuschließen,
eine Anzahl von verschlossenen Blutsaugrohren (a) wird in einer vorbestimmten Anordnung bzw. Matrix auf einem Anordnungstisch (3; 23) aufgereiht, zumindest eine Luftsaugnadel (5; 24) wird horizontal und vertikal bezüglich des Anordnungstisches (3; 23) bewegt, um ein vorderes Ende von dieser in das Innere von einem der Blutsaugrohre (a) einzuführen,
aus dem Blutsaugrohr (a) wird Luft gesaugt,
die Luftsaugnadel (5; 24) wird aus dem Blutsaugrohr (a) herausgezogen, während der Stopfen (b) des Blutsaugrohres gedrückt gehalten wird, und die Bewegungs-, Saug- und Herausziehschritte werden für jeden der bleibenden Blutsaugrohren wiederholt, wobei all die obigen Schritte durch eine Regeleinrichtung (18) automatisch ausgeführt und geregelt bzw. gesteuert werden.

5. Verfahren zum automatischen Evakuieren von Blutsaugrohren unter Verwendung der Vorrichtung gemäß Anspruch 3, mit den Schritten:
eine Anzahl von Blutsaugrohren (a) wird in einer Umgebung mit atmosphärischen Druck zum dichten Verschließen des Inneren der Blutsaugrohre mit Gummistopfen versehen,
eine Anzahl von mit Stopfen versehenen Blutsaugrohren (a) wird in einer vorbestimmten Anordnung bzw. Matrix auf einem Anordnungstisch (23) aufgereiht,
eine Vielzahl von Luftsaugnadeln (24) wird horizontal und vertikal bezüglich des Anordnungstisches (23) bewegt, um die vorderen Enden von diesen in das Innere der selben Anzahl von Blutsaugrohren (a) einzuführen,
Luft wird von den Saugrohren (a) gleichzeitig abgesaugt,
die Blutsaugnadeln (24) werden herausgezogen, während die Stopfen (b) der Blutsaugrohre (a) gedrückt gehalten werden und die Bewegungs-, Saug- und Herausziehschritte werden für eine Vielzahl von Sätzen wiederholt, von denen jeder aus der Vielzahl von Blutsaugrohren (a) besteht, wobei alle obigen Schritte durch eine Regeleinrichtung (18) automatisch ausgeführt geregelt bzw. gesteuert werden.

## Revendications

1. Dispositif destiné à mettre automatiquement sous vide des tubes pour prélèvement sanguin, comprenant :
une tablette d'agencement (3; 23) sur laquelle le nombre de tubes obturés pour prélèvement sanguin (a) à mettre sous vide sont disposés selon un pas prédéterminé,
au moins une aiguille d'aspiration d'air (5 ; 24) destinée à percer les bouchons ou obturateurs en caoutchouc des tubes de prélèvement et à expulser l'air de l'intérieur des tubes obturés pour prélèvement sanguin (a) pour la mise sous vide,
des moyens d'actionnement (6, 7, 8 ; 26, 27, 28) pour déplacer l'aiguille d'aspiration d'air (5 ; 24) horizontalement et verticalement par rapport à la tablette d'agencement (3 ; 23),
un élément poussoir (11 ; 29) destiné à comprimer les bouchons ou obturateurs en caoutchouc des tubes de prélèvement sanguin (a) pendant le mouvement ascendant vertical de l'aiguille d'aspiration d'air (5 ; 24) en dehors des tubes de prélèvement obturés,
une unité d'aspiration d'air (12) raccordée à l'aiguille d'aspiration (5 ; 24) pour aspirer l'air par cette aiguille d'aspiration d'air (5 ; 24), et
un tableau de commande (18) destiné à commander le fonctionnement de l'aiguille d'aspiration d'air (5 ; 24), des moyens d'actionnement (6, 7, 8 ; 26, 27, 28), un élément poussoir (11 ; 29) et l'unité d'aspiration d'air (12) destinée à expulser automatiquement l'air à partir de l'intérieur des tubes de prélèvement sanguin (a) maintenus dans un état de scellement hermétique.

2. Dispositif selon la revendication 1, comprenant de plus des moyens d'écoulement d'air comprimé (16, 17) en communication fluide avec l'aiguille d'aspiration d'air (5 ; 24) permettant d'assurer que l'air a traversé l'aiguille d'aspiration d'air (5 ; 24).

3. Dispositif destiné à mettre automatiquement sous vide des tubes de prélèvement sanguin selon la revendication 1, dans lequel la tablette d'agencement est fixe et plusieurs aiguilles d'aspiration d'air (24) destinées à expulser l'air depuis l'intérieur des tubes obturés de prélèvement sanguin (a) pour la mise sous vide, conjointement mobiles, et disposés de telle sorte que plusieurs tubes obturés de prélévement (a) peuvent être mis sous vide simultanément.

4. Procédé destiné à mettre automatiquement sous vide des tubes de prélèvement sanguin en utilisant le dispositif selon la revendication 1, comprenant les étapes consistant à :
obturer à l'aide d'un bouchon en caoutchouc un certain nombre de tubes de prélèvement sanguin (a) sous pression atmosphérique pour sceller hermétiquement l'intérieur des tubes de prélèvement sanguin,
disposer un certain nombre de tubes de prélèvement sanguin obturés (a) dans une disposition prédéterminée sur une tablette d'agencement (3 ; 23), déplacer au moins une aiguille d'aspiration d'air (5 ; 24) horizontalement et verticalement par rapport à la tablette d'agencement (3 ; 23) pour introduire une extrémité distale de celle-ci à l'intérieur de l'un des tubes de prélèvement sanguin (a),
aspirer l'air du tube de prélèvement sanguin (a),
extraire l'aiguille d'aspiration d'air (5 ; 24) hors du tube de prélèvement sanguin (a) tout en comprimant l'obturateur ou bouchon (b) du tube de prélèvement sanguin, et
répéter les étapes de déplacement, d'aspiration et d'extraction pour chacun des tubes de prélèvement sanguin restants, procédé dans lequel toutes les étapes précitées sont exécutées et commandées automatiquement par le tableau de commande (18).

5. Procédé destiné à mettre automatiquement sous vide les tubes de prélèvement sanguin en utilisant le dispositif de la revendication 3, comprenant les étapes consistant à :
obturer à l'aide d'un bouchon en caoutchouc un certain nombre de tubes de prélèvement sanguin (a) sous pression atmosphérique pour sceller hermétiquement l'intérieur des tubes de prélèvement sanguin,
agencer un certain nombre de tubes obturés de prélèvement sanguin (a) dans une disposition prédéterminée sur la tablette d'agencement (23),
déplacer plusieurs aiguilles d'aspiration d'air (24) horizontalement et verticalement par rapport à la tablette d'agencement (23) pour introduire ces extrémités distales à l'intérieur du même nombre de tubes de prélèvement sanguin (a),
aspirer simultanément l'air des tubes de prélèvement sanguin (a),
extraire les aiguilles de prélèvement sanguin (24) tout en comprimant les bouchons ou obturateurs (b) des tubes de prélèvement sanguin (a), et répéter les étapes de déplacement, d'aspiration et d'extraction pour une pluralité de séries, chacune étant constituée par une pluralité de tubes de prélèvement sanguin (a), procédé dans lequel toutes les étapes précitées sont exécutées et commandées automatiquement par le tableau de commande (18).
